# EUROPÄISCHE PATENTSCHRIFT

(11) **EP 3 151 776 B1**
(45) Veröffentlichungstag und Bekanntmachung des Hinweises auf die Patenterteilung: **07.11.2018**
(21) Anmeldenummer: 15730390.0
(22) Anmeldetag: 01.06.2015
(51) Int. Cl.: A61B 6/14, A61C 9/00, A61C 13/00, A61C 1/08

(54) **VERFAHREN ZUR HERSTELLUNG EINER ZAHNMEDIZINISCHEN BOHRSCHABLONE**
METHOD FOR PRODUCING A DENTAL DRILLING TEMPLATE
PROCÉDÉ DE FABRICATION D'UN GABARIT DE PERÇAGE À USAGE DENTAIRE

(30) Priorität: 03.06.2014 DE 102014007870
(43) Veröffentlichungstag der Anmeldung: 12.04.2017
(73) Patentinhaber: Med.dent.minds GmbH, 40668 Meerbusch (DE)
(72) Erfinder: SCHEFFER, Axel, 40668 Meerbusch (DE)
(74) Vertreter: Cohausz Hannig Borkowski Wißgott
(86) Internationale Anmeldenummer: PCT/EP2015/001110
(87) Internationale Veröffentlichungsnummer: WO 2015/185205

(56) Entgegenhaltungen:
- WO-A2-2008/045965
- DE-A1-102010 031 018
- FR-A1- 2 896 403

## Beschreibung

Die Erfindung betrifft ein Verfahren zur Herstellung einer zahnmedizinischen, an eine Kieferstruktur eines Patienten angepasste Bohrschablone mit wenigstens einem Leitkanal für die Führung eines Bohrwerkzeuges, bei dem der wenigstens eine Leitkanal in Abhängigkeit von Positionsdaten an einem einen Negativabdruck der Kieferstruktur aufweisenden Abdruckelement angeordnet wird.

Verfahren dieser Art sind im Stand der Technik bekannt und werden im Bereich der Implantologie eingesetzt. Im Wesentlichen beruhen diese Verfahren darauf, dass der Kiefer eines Patienten radiologisch erfasst und virtuell als Modell mit einer Datenverarbeitungsanlage visualisiert wird, wobei auf der Basis der visualisierten Daten die Position eines zukünftig zu setzenden Implantates und des dafür benötigten Leitkanales festgelegt wird, entlang dem mit einem Bohrwerkzeug eine Bohrung im Kieferknochen des Patienten gesetzt werden soll, um in die Bohrung ein Implantat einzubringen. Die Festlegung der Positionsdaten erfolgt dabei üblicherweise in Bezug zu einer radiologisch miterfassten Referenz, die an einem Abdruckelement vorhanden ist, das während der radiologischen Erfassung des Kiefers vom Patienten getragen wird. Beispielsweise mittels digitaler Volumentomographie kann eine Erfassung und anschließende Visualisierung des zu behandelnden Kiefers vorgenommen werden.

Die virtuelle Planung der Leitkanalpositionierung wird bereits im Stand der Technik vorteilhafterweise durchgeführt, um zum einen Bereiche mit genügend Knochenstruktur zu erschließen, zum anderen aber auch sicherzustellen, dass die für die Implantatsetzung vorgesehenen Bohrungen keine Nervenbahnen oder andere gefährdete Nachbarstrukturen verletzen.

Es wird somit aufgrund dieser bei der virtuellen Planung ermittelten Positionsdaten festgelegt, wo an einem Abdruckelement mit einem Negativabdruck der Kieferstruktur eines Patienten ein Leitkanal anzuordnen ist. Ein solches Abdruckelement mit wenigstens einem Leitkanal bildet eine Bohrschablone im Sinne der Erfindung, die auf eine vorhandene Kieferstruktur eines Patienten aufgesetzt werden, wonach ein Zahnarzt durch den wenigstens einen Leitkanal eine Bohrung vornehmen kann.
Das Dokument DE 10 2010 031 018 offenbart zum Beispiel ein solches Verfahren, bei welchem ein Negativabdruck der Kieferstruktur inklusive wenigstens einem Leitkanal als Ganzes aus einem Rohling computergestützt herausgearbeitet wird. Dieses im Stand der Technik bekannte Verfahren setzt jedoch aufwändige und teure Bearbeitungsmaschinen voraus, insbesondere solche, die in der Lage sind, Leitkanäle beispielsweise als Bohrung in dem Rohling in beliebigen Winkelorientierungen zu erstellen. Im Wesentlichen kann dies nur durch zeitaufwändige manuelle Justierprozeduren an mehrachsigen Montagesockeln durch erfahrenes Fachpersonal oder durch teure Fünf-Achsen-Bearbeitungsmaschinen erfolgen.
Die Erstellung solcher Bohrschablonen, die somit im Wesentlichen aus einem einen Negativabdruck der Kieferstruktur aufweisenden Abdruckelement und wenigstens einem Leitkanal gebildet sind, kann aus diesen Gründen üblicherweise nur durch spezialisierte Bearbeitungszentren vorgenommen werden. Dies bedeutet für die betroffenen Patienten, dass diese lange Wartezeiten in Kauf nehmen müssen, weil die eigenen Zahnärzte die Anfertigung solcher Bohrschablonen nicht selbst vornehmen.

Es ist daher eine Aufgabe der Erfindung, ein Verfahren bereitzustellen, mit dem die Anfertigung solcher Bohrschablonen zur Unterstützung beim Setzen von Implantaten auf einfache, schnelle und kostengünstige Art und Weise erfolgen kann, wodurch sich Vorteile für den Patienten erschließen, nämlich geringere Wartezeiten sowie auch Vorteile für die behandelnden Zahnärzte, nämlich die Erschließung zusätzlicher Betätigungsfelder und daraus resultierender Einnahmen.

Insbesondere ist es auch eine Aufgabe der Erfindung, ein Verfahren bereitzustellen, das die beliebige Positionierung von Leitkanälen an oder in einem einen Negativabdruck der Kieferstruktur aufweisenden Abdruckelement erlaubt unter Zuhilfenahme weniger aufwendiger und teuren Bearbeitungsmaschinen, wie beispielsweise Vier-Achsen-Maschinen, sowie unter Anwendung bereits bekannter Arbeitsschritte.

Die Aufgabe wird gemäß der Erfindung dadurch gelöst, dass das eingangs genannte, gattungsgemäße Verfahren weitergebildet wird durch die Schritte, dass ein ein Passungsstück aufweisendes, an die benötigte Position des Leitkanales angepasstes Montageelement durch Materialbearbeitung mittels einer Bearbeitungsmaschine gefertigt wird, wobei während der Fertigung in Abhängigkeit der Positionsdaten eine formschließende Montagestruktur, insbesondere in wenigstens zwei Richtungen periodische formschließende Montagestruktur am Montageelement erzeugt wird und weiterhin ein den Leitkanal, insbesondere in Form einer Metallhülse bereits umfassendes Kanalelement mit einem zum Passungsstück des Montageelementes korrespondierenden Passungsbereich, insbesondere der durch den Leitkanal selbst gebildet wird, an dem gefertigten Montageelement befestigt wird, wonach die Paarung von Montagelement und Kanalelement mit der formschließenden Montagestruktur des Montagelementes an einer in Abhängigkeit der Positionsdaten berechneten Stelle an einer eine korrespondierende formschließende Struktur, bevorzugt in wenigstens zwei Richtungen periodische Struktur aufweisende Montageplatte befestigt wird, gegenüberliegend zur Montageplatte mit einem Befestigungselement das Abdruckelement befestigt wird und das Kanalelement durch Befestigung, insbesondere durch klebende Befestigung, am Abdruckelement und Lösung vom Montageelement an dem Abdruckelement angeordnet wird.

Unter einer formschließenden Montagestruktur wird eine Strukturierung einer Fläche am Montageelement verstanden, die bei Anbringung des Montageelementes an der Montageplatte mit der dortigen korrespondierenden , bevorzugt ebenso flächigen Struktur einen Formschluss eingeht, so dass hierdurch nach der Befestigung dieser Teile aneinander die Position des Montageelementes an der Montageplatte, insbesondere die Ausrichtung von dessen Passungsstück sicher fixiert ist. Die Struktur an dem Montageelement und der Montageplatte kann bevorzugt in wenigstens zwei Richtungen (parallel zur Ebene der Fläche, die die Struktur aufweist) periodisch sein. Insbesondere kann die Montagestruktur am Montageelement zur Struktur an der Montageplatte identisch sein. Die Montagestruktur und Struktur an der Montageplatte können, besonders bei Identität zueinander so ausgebildet sein, dass eine invertierte Form / Negativform der Struktur wiederum der Struktur selbst entspricht, somit also auch eine Inversionsidentität vorliegt.

Das erfindungsgemäße Verfahren macht sich hier besonders zu Nutze, dass ein Leitkanal, der zur späteren Führung eines Bohrwerkzeuges in bzw. an der Bohrschablone vorgesehen ist, nicht selbst mittels einer Bearbeitungsmaschine erstellt werden muss, sondern ein Rückgriff auf vorgefertigte oder einfach herzustellende Elemente erfolgt. Bereits hierdurch werden Kosten und Arbeitszeiten eingespart.

Bearbeitungsmaschinen für den Einsatz des erfindungsgemäßen Verfahrens können weiterhin mit weniger Freiheitsgraden auskommen, als eine solche, die gemäß Stand der Technik zur Herstellung von Leitkanälen in beliebigen Winkelausrichtungen benötigt wird. Insbesondere können nicht nur subtraktiv arbeitende Bearbeitungsmaschinen, wie z.B. Fräsen, sondern auch additive / generative Bearbeitungsmaschinen eingesetzt werden, wie z.B. 3D-Drucker.

Erfindungsgemäß ist es hier vorgesehen, dass ein Kanalelement eingesetzt wird, welches bereits einen vorgefertigten Leitkanal, zum Beispiel in der Form einer Metallhülse aufweist, die z.B. von einem Kunststoffmaterial umgeben sein kann, wobei sodann gemäß den vorbeschriebenen Schritten ein solches Kanalelement in der richtigen Positionierung mittels Montageelement und Montageplatte in die benötigte Lage relativ zum Abdruckelement gebracht wird, um sodann das Kanalelement auf das Abdruckelement zu übertragen, nämlich dadurch, dass es am Abdruckelement befestigt wird, beispielsweise durch Kleben, und die vorherige Befestigung zum Montageelement gelöst wird.

Die benötigte Position und Orientierung bzw. Achsausrichtung des Leitkanales wird dabei sichergestellt durch die Orientierung der Montagestruktur des Montageelementes, welches das Kanalelement über die Passung definiert trägt und die Befestigung des Montageelementes über seine formschließende daran angeordnete Montagestruktur an der korrespondierenden Struktur der Montageplatte. Hierzu wird die Montagestruktur in der benötigten relativen Lage zum Passungsstück am Montageelement gefertigt, insbesondere was in Abhängigkeit der Positionsdaten erfolgt, die die benötigte Position eines Leitkanales definieren.

Die Fertigung eines Montageelementes kann z.B. materialabtragend erfolgen. Z.B. kann ein Montageelement-Rohling vorgesehen sein, beispielsweise als Kunststoffblock der bereits ein Passungsstück aufweist und der materialabtragend so bearbeitet wird, dass die benötigte Montagestruktur entsteht, also aus dem Rohling herausgearbeitet wird. Hierfür kann ein Montageelement-Rohling weiterhin auch ein Spannelement aufweisen, mit dem der Montageelementrohling in einer Bearbeitungsmaschine eingespannt werden kann.

Alternativ besteht auch die Möglichkeit, ein Montageelement mittels einer additiven/generativen Fertigungstechnik herzustellen, z.B. mittels Materialauftrag durch 3D-Druck. Hier können sowohl Kunststoff- als auch Metallmaterialien Verwendung finden. Sowohl die Montagestruktur, also auch das Passungsstück und ein diese Bereiche verbindender Bereich kann generativ als ein Bauteil hergestellt werden.

Zusätzlich zu dem erfindungsgemäßen Schritt, dass das Montageelement patientenindividuell gefertigt wird, kann es auch vorgesehen sein, dass ein Kanalelement ebenso patientenindividuell gefertigt wird, z.B. aus einem den Leitkanal bereits umfassenden Kanalelement-Rohling gefertigt wird, insbesondere ebenso in Abhängigkeit der Positionsdaten und beispielsweise wiederum durch Materialabtrag in einer Bearbeitungsmaschine.

Hierbei kann es erfindungsgemäß vorgesehen sein, dass die Bearbeitung eines Kanalelement-Rohlings und/oder Montageelement-Rohlings mit derselben Bearbeitungsmaschine, z.B. einer Vier-Achsen-Fräse erfolgt, in welcher ein jeweiliger Rohling mittels eines am Rohling angeordneten Spannelementes eingespannt wird.

Ebenso kann auch hier ein Kanalelement mittels generativer Fertigung hergestellt werden, z.B. durch sogenannten 3D-Druck aus Kunststoff oder Metall. Es kann hierbei vorgesehen sein, dass der Leitkanal so wie das gesamte Kanalelement generativ erstellt wird, insbesondere was dann bevorzugt in metallischer Ausführung erfolgt. Ebenso kann eine vorhandene Metallhülse durch generative Fertigung mit Kunststoff in einer Bearbeitungsmaschine umhüllt werden.

Zwecks Bestimmung von Positionsdaten kann es, wie im Stand der Technik bekannt, vorgesehen sein, dass der Kiefer eines zu behandelnden Patienten radiologisch, beispielsweise durch digitale Volumentomographie erfasst wird. Die Erfindung kann es dafür vorsehen, dass ein Abdruckelement mit einem Negativabdruck der Kieferstruktur des zu behandelnden Patienten angefertigt wird, welches später Teil der herzustellenden Bohrschablone ist.

Hier ist es ein weiterer Vorteil, z.B. gegenüber dem eingangs genannten Stand der Technik, dass für die Erstellung eines Abdruckelementes auf bekannte kostengünstige Techniken zurückgegriffen werden kann, z.B. die Abformung einer Kieferstruktur mittels einer Abdruckmasse erfolgt oder der Abformung von einem Gipsmodell des Kiefers mittels einer Tiefziehfolie, insbesondere wobei Hinterschneidungen am Gebiss des Patienten oder am Modell vor Applikation der Masse oder der Tiefziehfolie aufgefüllt werden.

Erfindungsgemäß kann ein bereits anfänglich, schon für die radiologische Erfassung verwendetes Abdruckelement zu einem späteren Bestandteil der erfindungsgemäßen Bohrschablone werden, indem genau an diesem Abdruckelement wenigstens ein Kanalelement befestigt wird.

Die Erfindung sieht weiterhin vor, dass das Abdruckelement mit einer radiologischen Referenz, insbesondere einer Röntgenreferenz versehen wird, um nach Positionierung des Abdruckelementes im bzw. am Kieferbereich des Patienten radiologisch in Verbindung mit einer Referenz für die Positionsdaten erfasst wird.

Es kann sodann softwarebasiert in einer virtuellen Darstellung der Kieferstruktur des Patienten die Position eines Leitkanales festgelegt werden, wobei dies in relativem Bezug zu der Referenz erfolgt, die an dem Abdruckelement befestigt ist. In Abhängigkeit der so gefundenen Positionsdaten erfolgt die Fertigung von Montageelement und/oder Kanalelement.

Die Erfindung kann in bevorzugter Weise vorsehen, dass das Abdruckelement zum Zweck der Übertragung des Kanalelementes auf dieses mit einem solchen Befestigungselement an der Montageplatte direkt oder indirekt über Hilfsmittel befestigt wird, welches gleichzeitig eine radiologische Referenz, insbesondere Röntgenreferenz umfasst.

So kann ein solches Befestigungselement ortsfest am Abdruckelement vorgesehen sein und somit sowohl für die radiologische Erfassung des Kiefers des Patienten eingesetzt werden als auch für die spätere Befestigung direkt oder indirekt über Hilfsmittel an der Montageplatte, wobei gleichzeitig sichergestellt wird, dass durch die virtuelle relative Positionierung des Leitkanales in Relation zur radiologischen Referenz automatisch auch eine Positionierung in Relation zur Montageplatte erfolgt, sofern die Lage der Montageplatte zur radiologischen Referenz bekannt und fest ist.

Um dies sicherzustellen, kann es vorgesehen sein, dass das Befestigungselement mit der Montageplatte oder daran befestigten Hilfsmitteln in reproduzierbaren Eingriff gebracht werden kann.

Beispielsweise kann eine Befestigung des Abdruckelementes mittels des Befestigungselementes an der Montageplatte oder einem daran befestigten Hilfsmittel dadurch erfolgen, dass das Befestigungselement Metallelemente, beispielsweise Metallstifte, aufweist, die in korrespondierende Ausnehmungen bzw. Bohrungen an der Montageplatte bzw. daran angeordnetem Hilfsmittel eingesteckt werden können. Ein Hilfsmittel kann z.B. ein flächiges Element sein, dass in einem Abstand parallel zur Montageplatte an dieser befestigt ist, insbesondere wobei das flächige Element eine Ausnehmung aufweist, insbesondere hierdurch die Form eines flächigen Rahmens ausbildet.

Hier können die Metallelemente, insbesondere die Metallstifte, die radiologische Referenz ausbilden. Das Befestigungselement kann z.B. als vestibulärer Bogen ausgebildet sein, der das Ausdruckelement auf dessen Außenseite umgibt.

Die Erfindung umfasst auch die Möglichkeit die Relation des Modells zu der Montageplatte über eine Rahmenkonstruktion wiederzugeben. Dies ist vorteilhaft, da dann das parallel zur Montageplatte im Abstand angeordnete flächige Hilfsmittel und/oder die vestibulär angebrachte Röntgenreferenz ohne Verlust der Orientierung abgenommen werden kann. So werden auch die durch dieses Hilfsmittel überdeckten Flächen der Montageplatte für die Montage der Montageelemente freigegeben. Gleichzeitig ist auch das Modell zur Überprüfung lagerichtig zugeordnet. Zur Herstellung dieser Rahmenkonstruktion wird der Bezug zuerst über die oben beschriebene Weise hergestellt und dann die Montageplatte durch eine Montageplatte der Rahmenkonstuktion, die gleichzeitig den abnehmbaren oberen Teil der Rahmenkonstruktion bildet, getauscht und ein individueller Gipssockel für die lagerichtige Position des Modells auf der Grundplatte hergestellt.

Die Erfindung kann vorsehen, dass mittels radiologischer Bilderfassung, insbesondere mittels tomographischer Bildgebungstechnik wie beispielsweise digitaler Volumentomographie, softwarebasiert ein virtuelles Kiefermodell eines Patienten erzeugt wird und ein virtueller Leitkanal relativ zum virtuellen Kiefermodell und zu der Referenz positioniert wird, wobei aus den ermittelten Positionsdaten des wenigstens einen virtuellen Leitkanals solche Positionsdaten gebildet werden, welche die Position der formschließenden Montagestruktur am Montageelement bestimmen. Sodann kann anhand dieser die Position der formschließenden Montagestruktur am Montageelement bestimmenden Positionsdaten mit einer Bearbeitungsmaschine das Montageelement mit der benötigten Lage der Montagestruktur gefertigt werden, z.B. aus einem Rohling.

Eine besonders bevorzugte Ausführung des erfindungsgemäßen Verfahrens kann es vorsehen, dass die formschließende Montagestruktur am Montageelement und/oder die formschließende Struktur an der Montageplatte als sich verjüngende in wenigstens zwei Richtungen periodisch nebeneinander angeordnete Vorsprünge ausgebildet ist.
Solche Vorsprünge können beispielsweise als pyramidenförmige Vorsprünge mit wenigstens drei, bevorzugt vier zueinander geneigten Seitenflächen ausgebildet sein, insbesondere deren Grundseiten einander berühren.

Auch können solche Vorsprünge als kegelförmige Vorsprünge ausgefertigt werden.

Eine solche Wahl der formschließenden Montagestruktur / Struktur ist hier besonders bei der abtragenden Fertigung aus Rohlingen vorteilhaft, da solche Strukturen selbst mit einer einfachen, lediglich vier Achsen aufweisenden Bearbeitungsmaschine in Ebenen angeordnet werden können, die verschiedenste Winkel relativ zu den Erstreckungsachsen von Leitkanal bzw. Passstück aufweisen.

Dabei wird unter einer Ebene, in der die Vorsprünge angeordnet sind, eine solche Ebene verstanden, in welcher gleiche Bereiche aller Vorsprünge, also beispielsweise alle Spitzen der Vorsprünge oder alle Grundseiten der Vorsprünge angeordnet sind.

Durch die genannte Wahl der Vorsprünge kann hier sichergestellt werden, dass selbst mit einer einfacheren Vier-Achsen-Maschine Ebenen, in denen die Vorsprünge liegen, unter einer Vielzahl von Winkeln, beispielsweise plus / minus 45 Grad zu einer Bearbeitungsachse der Maschine erstellt werden können, da durch die verjüngende Ausführung der Vorsprünge in die Richtung zu einem Bearbeitungswerkzeug, bezogen auf dieses Werkzeug keine Hinterschnitte in der formschließenden Struktur existieren.

Um hier einen möglichst großen Winkelbereich zu erschließen, kann es vorgesehen sein, dass die Seitenflächen von pyramidalen oder kegeligen Vorsprüngen relativ zu ihrer Basisfläche bzw. der vorgenannten Ebene, in welcher die Vorsprünge angeordnet sind, unter 45 Grad liegen. Sodann kann ohne Erzeugung von Hinterschnitten die Ebene, in welcher die Vorsprünge angeordnet sind, um bis zu 45 Grad zu einer Bearbeitungsachse der Bearbeitungsmaschine geneigt sein, insbesondere zu der Bearbeitungsachse, in deren Richtung ein Werkzeuge der Maschine auf einen Rohling zu bzw. zurück bewegt wird.

Die Erfindung erschließt es hierdurch, dass selbst mit Bearbeitungsmaschinen, die lediglich vier oder gar nur drei Achsen aufweisen, solche Positionierungen von Leitkanälen erfolgen können, die bislang nach dem Stand der Technik lediglich mit komplizierteren Fünf-Achs-Maschinen ermöglicht wurden.

Die Erfindung hat weiterhin den Vorteil, dass ein behandelnder Zahnarzt z.B. auf vorgefertigte Rohlinge zurückgreifen kann, die durch eine Bearbeitungsmaschine lediglich in eine benötigte Form gebracht werden müssen, was vor Ort in der Zahnarztpraxis sehr schnell erfolgen kann.

So kann die Erfindung hierfür beispielsweise vorsehen, dass ein Montageelement-Rohling ein Block aus einem beispielsweise spanend abtragbaren Material, wie zum Beispiel einen Kunststoff umfasst, wobei an bzw. in dem Block ein Passungsstück angeordnet ist. Ebenso kann die Erfindung ein Kanalelemente-Rohling vorsehen, der einen Block aus einem insbesondere spanend abtragbaren Material wie beispielsweise einen Kunststoff umfasst, wobei an bzw. in dem Block ein Leitkanal, beispielsweise in der Art einer Metallhülse ausgebildet ist, insbesondere wobei weiterhin ein solcher Kanalelement-Rohling auch einen Passungsbereich aufweist für die Verbindung mit dem vorgenannten Montageelement-Rohling bzw. dessen Passstück, wobei dieser Passungsbereich beispielsweise durch den Leitkanal selbst gebildet werden kann.

Die Erfindung kann auch weiterhin vorsehen, dass ein Kanalelement-Rohling in einer Stirnfläche des Leitkanales oder um den Leitkanal herum Orientierungselemente, wie beispielsweise Kerben aufweist. Beispielsweise dann, wenn ein Passungsstück eines Montageelement-Rohlings korrespondierende Stege, zum Beispiel um einen Passungsstift herum, aufweist, kann eine definierte Winkellage zwischen Kanalelement-Rohling und Montageelement-Rohling bei deren Aneinandermontage erzielt werden. Die vorgenannten Kerben an einem Kanalelement-Rohling können jedoch auch weiterhin dafür eingesetzt werden, um den Schaft eines späteren Implantates in einer vordefinierten Winkelposition bezogen auf seine Rotation in den Kieferknochen einzudrehen.

Die Erfindung kann auch vorsehen, dass es ein Set von Rohlingen der vorgenannten Arten gibt, bei denen die relative Lage zwischen einem Passungsstück bzw. einem Leitkanal zu einem Spannelement für die Befestigung des Rohlings einer Bearbeitungsmaschine unterschiedlich ist. Je nach Anforderung bzw. benötigter Positionierung des Leitkanales kann es hier sinnvoll sein, auf unterschiedlich vorgefertigte Rohlinge mit den verschiedenen Winkellagen relativ zum Spannelement zurückgreifen zu können.

Ein Ausführungsbeispiel der Erfindung wird anhand der nachfolgenden Figuren näher beschrieben.

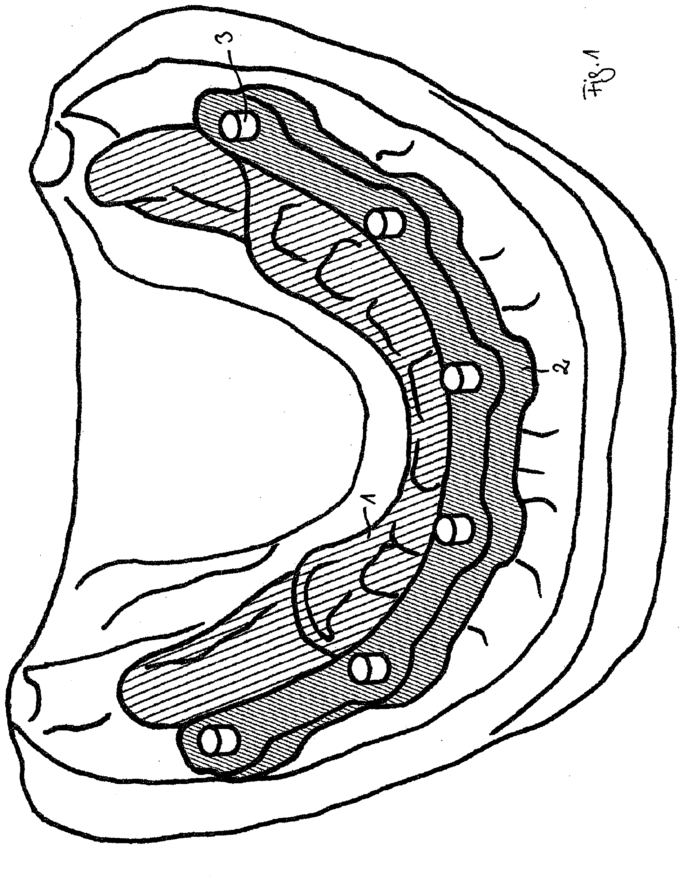

Die Figur 1 zeigt in Aufsicht ein Abdruckelement 1, das einen Negativabdruck einer Kieferstruktur eines Patienten aufweist. Im vorliegenden Fall ist das Abdruckelement 1 an einem Gipsmodell der Kieferstruktur eines Patienten angebracht. Es besteht selbstverständlich die Möglichkeit, das Abdruckelement 1 auch konkret am Kiefer des Patienten zu befestigen, beispielsweise um die radiologische Erfassung des Kiefers vorzunehmen.

Hierfür ist an dem Abdruckelement 1, das zum Beispiel als eine Tiefzieh-Folie oder auch als erhärtete Abdruckmasse ausgebildet sein kann, eine radiologische Referenz, z.B. Röntgenreferenz 2 angeordnet, hier im vestibulären Bereich als Bogen, wobei diese Referenz 2 hier röntgendichte Metallstifte 3 aufweist, die sich von der Oberfläche des bogenförmigen Elementes nach oben erstrecken.

Die bogenförmige Referenz 2 mit den Metallstiften 3 bildet hier erfindungsgemäß auch ein Befestigungselement 2 für die spätere Montage an der Montageplatte, bzw. einem daran angeordneten Hilfsmittel, wie nachfolgend noch beschrieben wird.

Die Erfindung kann hier in üblicher Weise vorsehen, dass durch radiologische Erfassung, beispielsweise durch digitale Volumentomographie, der Kiefer des Patienten virtuell visualisiert werden kann, um den Zahnarzt in die Lage zu versetzen, anhand der örtlichen Gegebenheiten des Kieferknochens für ein zu setzendes Implantat einen Leitkanal zu definieren, durch den hindurch später ein Bohrwerkzeug geführt wird.

Erfindungsgemäß ist vorgesehen, einen solchen Leitkanal an dem Abdruckelement 1 zu befestigen und hierdurch insgesamt eine Bohrschablone im Sinne der Erfindung auszubilden.

Selbstverständlich kann die Erfindung vorsehen, nicht nur einen einzigen Leitkanal an einem solchen Abdruckelement 1 anzuordnen, sondern es können grundsätzlich mehrere, gegebenenfalls beliebige viele Leitkanäle vorgesehen werden, insbesondere da gemäß der Erfindung keine tragenden Zahnstrukturen benötigt werden.

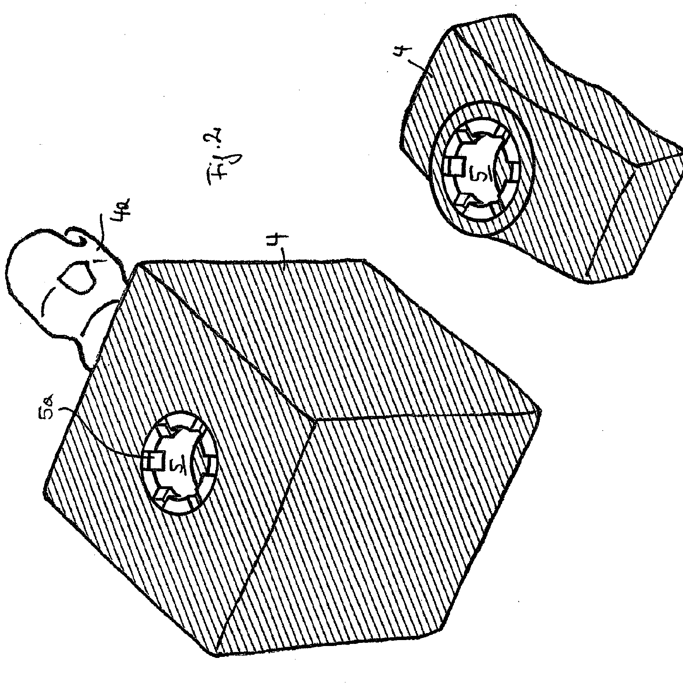

Die Figur 2 zeigt linksseitig einen Kanalelement 4 als Rohling in der Form eines Blockes, beispielsweise eines würfelförmigen oder quaderformigen Blockes aus einem bearbeitbaren, zum Beispiel spanend bearbeitbaren Material, wie Kunststoff. Der Block weist an einer seiner Seitenflächen ein Spannelement 4a auf, mit dem der Block in einer Bearbeitungsmaschine für die vorgesehene Bearbeitung eingespannt werden kann.

Erfindungswesentlich ist es hier vorgesehen, dass der Rohling des Kanalelements 4 in einer seiner Seitenflächen einen Leitkanal 5 aufweist, der zum Beispiel durch eine Metallhülse ausgebildet ist, die sich von der Oberfläche des Kanalelements 4 / Rohlings in die Tiefe erstreckt.

Hier ist weiterhin in der Figur 2 dargestellt, dass der hülsenförmige Leitkanal 5 um die Mittenachse herum in seiner Stirnfläche in gleichmäßiger Winkelteilung angeordnete Kerben 5a aufweist. Die Figur 2 zeigt rechtsseitig ein fertig bearbeitetes Kanalelement 4 nach spanender Bearbeitung durch eine Bearbeitungsmaschine.

Dies kann vorgesehen sein, um überschüssiges Material aus dem Rohling zu entfernen, beispielsweise wenn dies die Platzverhältnisse im Kieferbereich erfordern oder um die Lage der Stirnfläche des Kanals 5 exakt gewünscht relativ zum Abdruckelement festzulegen und so die spätere Bohrtiefe zu definieren.

Die Erfindung kann grundsätzlich auch vorsehen, Kanalelemente 4 einzusetzen, die vor der weiteren erfindungsgemäßen Befestigung an dem Abdruckelement mittels eines nachfolgend noch zu beschreibenden Montageelementes keinerlei Bearbeitung erfahren müssen.

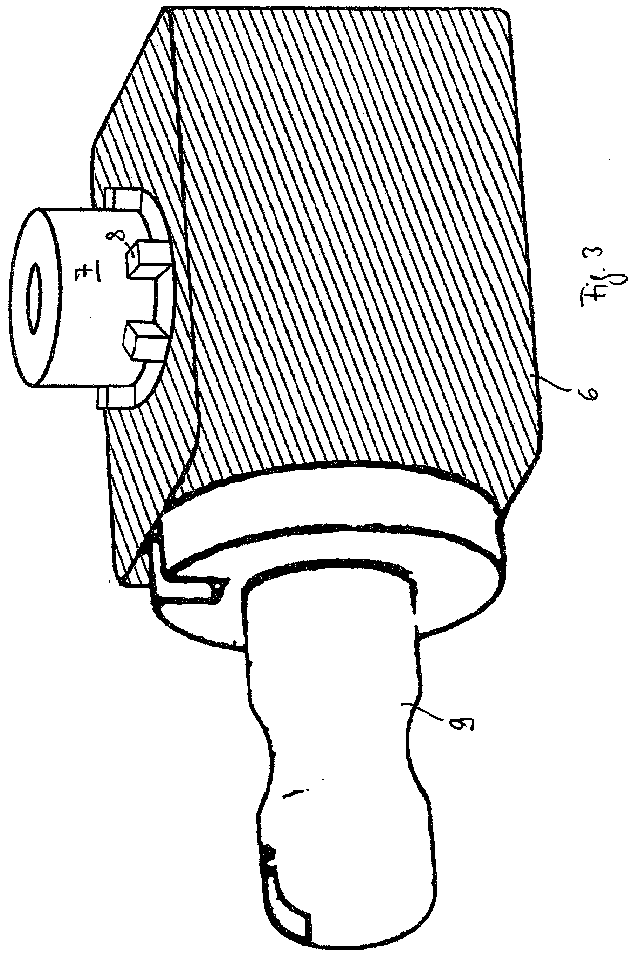

Die Figur 3 zeigt in einer Übersichtsdarstellung einen Rohling eines Montageelements 6 mit im Wesentlichen quader- oder auch würfelförmiger Ausgestaltung, wobei auf einer der Seitenflächen des Rohlings ein Passungsstück 7 vorspringend angeordnet ist. Dieses Passungsstück 7 ist an den Leitkanal 5 des Kanalelementes angepasst, wobei hier ergänzend um das Passungsstück 7 auch zu den Kerben 5a korrespondierende Stege 8 vorgesehen sind. Unter Anpassung wird in diesem Beispiel verstanden, dass das Passungsstück 7 in den Leitkanal 5 im Wesentlichen spielfrei eingesteckt werden kann. Es besteht so die Möglichkeit, ein Kanalelement 4 in definierter Lage an einem Montageelement-Rohling 6, insbesondere nach dessen Bearbeitung anzubringen.

Der hier dargestellte Rohling 6 weist ebenso, wie in der Figur 2 zum Kanalelement beschrieben, ein Spannelement 9 auf, mit dem ein solcher Rohling in einer Bearbeitungsmaschine aufgenommen werden kann. Es kann vorgesehen sein, Rohlinge mit verschiedenen Orientierungen zwischen Spannelement 9 und Passungsstück 7 einzusetzen.

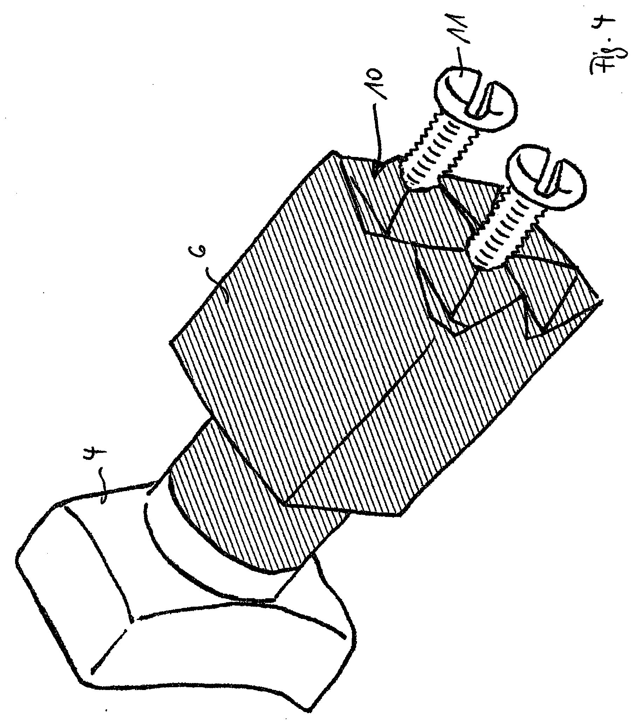

Gemäß der Erfindung ist es nun vorgesehen, so wie es die Figur 4 zeigt, dass in Abhängigkeit von ermittelten Positionsdaten für den Leitkanal 5 aus dem Montageelement-Rohling ein Montageelement 6 gefertigt wird, welches eine ' formschließende Montagestruktur 10 aufweist, die im hier vorliegenden Fall als pyramidenförmige Vorsprünge mit vier gleichen Seitenflächen ausgebildet ist. Die Figur 4 zeigt, dass in diese Struktur Gewindebohrungen mit Schrauben 11 eingebracht werden können, die einer späteren Befestigung dienen. An dem Montageelement 6 ist gemäß der Figur 4 das Kanalelement 4 befestigt, dadurch dass das Passstück 7 des Montageelementes 6 in den Leitkanal 5 eingesteckt ist. Hierdurch bilden Kanalelement 4 und Montageelement 6 eine Bauteil-Paarung.

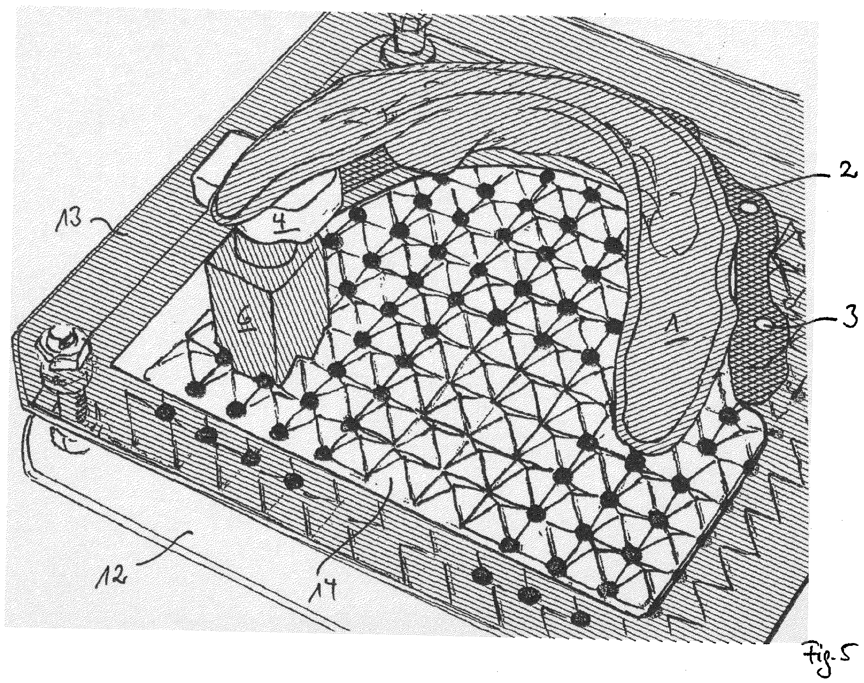

Die formschließende Montagestruktur 10 wird hinsichtlich der Lage der in dieser Ausführung pyramidalen Vorsprünge und der Ebene, in der diese Vorsprünge, insbesondere deren Spitzen oder deren Grundseiten liegen, derart an dem Montageelement angeordnet, dass dieses Montageelement 6 nach einer Befestigung an einer vorbestimmten Stelle an einer Montageplatte 12, wie sie die Figur 5 zeigt, den Leitkanal 5 des Kanalelementes 4 in der korrekten benötigten Lage relativ zum Abdruckelement 1 positioniert, das mittels den Metallstiften 3 an der Montageplatte 12, hier indirekt über ein dazu parallel im Abstand angeordnetes flächiges Hilfsmittel 13 befestigt wird.

Hier weist das flächige, bevorzugt rahmenförmige Hilfsmittel 13 eine Reihe von nicht dargestellten Bohrungen auf, die mit der Position der Metallstifte 3 korrespondieren, so dass das Abdruckelement 1 mittels des Befestigungselementes 2 in einer Gegenüberlage mit Abstand zur Montageplatte 12 befestigt werden kann.

Das Montageelement 6 ist mit seiner daran gefertigten Montagestruktur 10 in formschließendem Eingriff mit einer korrespondierenden Struktur 14, die auf der Montageplatte 12 angeordnet ist und ebenso in dieser Ausführung pyramidale Vorsprünge ausbildet. Die Vorsprünge sind hier ersichtlich in zwei Richtungen periodisch nebeneinander angeordnet, insbesondere so, dass deren Grundseiten sich jeweils berühren.

Die pyramidalen Vorsprünge der Montagestruktur am Montageelement 6 und der Struktur 14 an der Montageplatte sind hier zueinander identisch ausgebildet, so dass im Raster der Periodizität ein Montageelement 6 mit der daran angeordneten maschinell hergestellten Montagestruktur an der Struktur 14 der Montageplatte formschließend befestigt werden kann.

Die für die korrekte Lage des Leitkanales benötigte Position des Montageelementes auf der Struktur der Montageplatte kann zum Beispiel durch eine Software im Raster der Periodizität berechnet und dem Nutzer ausgegeben werden, so dass der behandelnde Zahnarzt die korrekte Position für die Montage auf der Platte auffindet. Die Platte 12 kann in der Struktur 14 eine Vielzahl von Bohrungen aufweisen, durch welche hindurch die in der Figur 4 dargestellten Schrauben 11 von der Unterseite der Montageplatte 12 in den Montageblock eingeschraubt werden können, um diesen sicher in der Struktur 14 zu befestigen.

Die Figur 5 zeigt, dass das Kanalelement 4 eine relative Position zum Abdruckelement 1 einnimmt, die durch die vorherige Positionsbestimmung des virtuellen Leitkanales und die Fertigung des Montageelementes 6 vorbestimmt wurde.

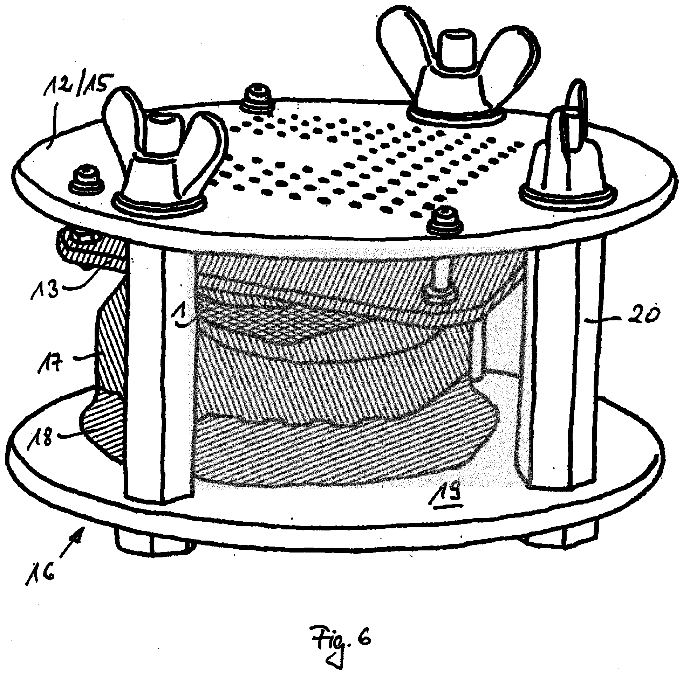

Alternativ kann eine Zuordnung der Montageplatte 12 zum Abdruckelement über eine Rahmenkonstruktion 16 erfolgen, die in Figur 6 dargestellt ist, wobei das Hilfsmittel 13 und die Röntgenreferenz mit Metallstiften 3 für die Zuordnung entbehrlich werden, insbesondere entfernt werden können. Die Rahmenkonstruktion umfasst eine Bodenplatte 19 und eine Deckelplatte 15, die durch Abstandselemente 20 in einem gewünschten Abstand und bevorzugt parallel zueinander gehalten werden.

Für die Montage eines Modells 17 der Kiefers, z.B. mittels eines Sockels 18 auf einer Bodenplatte 19 der Rahmenkonstruktion 16 kann die eingangs genannte Montageplatte 12 gegen eine Deckelplatte 15 der Rahmenkonstruktion getauscht werden. Hier weist die Deckelplatte 15 sodann dieselbe formschließende und lagegleiche Struktur auf seiner Unterseite auf, wie die zuvor beschriebene Montageplatte 12. Das Hilfselement 13 ist in gleicher Weise und lagegleich an der Deckelplatte 15 befestigbar wie an der Montageplatte 12. Insbesondere kann die zuvor beschriebene Montageplatte 12 so ausgebildet sein, dass diese der Deckelplatte 15 gleichkommt, somit also ein Austausch entfällt.

Nach der Positionierung des Modells 17 mit aufgesetztem Abdruckelement 1 auf der Bodenplatte mittels des Befestigungselementes an dem Abdruckelement und dessen Eingriff in das Hilfselement 13 kann das Hilfselement 13 entfernt werden. Insbesondere sofern die Positionierung des Modells auf der Bodenplatte fixiert ist.

Mit Bezug auf Figur 5 und alternativ bei der Ausführung der Figur 6 besteht nun die Möglichkeit, das Kanalelement 4 mit dem Leitkanal 5 z.B. klebend an dem Abdruckelement 1 zu befestigen, so dass aus der dann gebildeten Kombination aus Leitkanal-Element 4 und Abdruckelement 1 die erfindungsgemäß herzustellende Bohrschablone gebildet ist. Hierfür wird das Kanalelement 4 vom Montageblock 6 sodann getrennt.

Der behandelnde Zahnarzt kann einem zu behandelnden Patienten das Abdruckelement 1 einsetzen, wobei sodann automatisch für den Zahnarzt der Leitkanal zur Bohrerführung zum Setzen eines Implantatschaftes zugänglich ist.

Die Erfindung kann beispielsweise weiterhin auch vorsehen, dass mittels einer Bearbeitungsmaschine in einen Rohling eines Leitkanalelementes zum Beispiel auch Bohrungen zur Kühlung während des Bohrvorganges eingebracht werden. Solche Kühl-Bohrungen können z.B. den Leitkanal schneiden.

Besonders die Figur 5 zeigt deutlich, dass entlang der bogenförmigen Erstreckung des Abdruckelementes 1 grundsätzlich beliebig viele Kanalelemente 4 platziert werden können.

Hier ist auch als weiterer Vorteil erkennbar, dass für die Platzierung von Leitkanälen keine Tragstrukturen in Form noch vorhandenen Zähnen an einer Kieferstruktur ausgebildet sein müssen. Das erfindungsgemäße Verfahren ist daher zum Beispiel auch geeignet, völlig zahnlose Kiefer oder zahnreduzierte Kieferabschnitte mit Implantaten zu versehen.

## Patentansprüche

1. Verfahren zur Herstellung einer zahnmedizinischen an eine Kieferstruktur eines Patienten angepasste Bohrschablone mit wenigstens einem Leitkanal (5) für die Führung eines Bohrwerkzeuges, bei dem der Leitkanal (5) in Abhängigkeit von Positionsdaten an einem einen Negativabdruck der Kieferstruktur aufweisenden Abdruckelement (1) angeordnet wird, wobei
a. ein ein Passungsstück (7) aufweisendes, an die benötigte Position des Leitkanales (5) angepasstes Montageelement (6) durch Materialbearbeitung mittels einer Bearbeitungsmaschine gefertigt wird
b. wobei während der Fertigung in Abhängigkeit der Positionsdaten eine formschließende Montagestruktur (10), bevorzugt in wenigstens zwei Richtungen periodische formschließende Montagestruktur (10) am Montageelement (6) erzeugt wird,
c. ein den Leitkanal (5), insbesondere in Form einer Metallhülse bereits umfassendes Kanalelement (4) mit einem zum Passungsstück (7) des Montageelementes (6) korrespondierenden Passungsbereich, insbesondere der durch den Leitkanal (5) selbst gebildet wird, an dem gefertigten Montageelement (6) befestigt wird,
d. die miteinander befestigte Paarung von Montageelement (6) und Kanalelement (4) mit der formschließenden Montagestruktur (10) des Montageelementes (6) an einer in Abhängigkeit der Positionsdaten berechneten Stelle an einer eine korrespondierende formschließende Struktur (14), insbesondere in wenigstens zwei Richtungen periodische Struktur (14) aufweisenden Montageplatte (12) befestigt wird,
e. gegenüberliegend zur Montageplatte (12) mit einem Befestigungselement (2) das Abdruckelement (1) befestigt wird und
f. das Kanalelement (4) durch Befestigung, insbesondere klebende Befestigung am Abdruckelement (1) und Lösung vom Montageelement (6) an dem Abdruckelement (1) angeordnet wird.

2. Verfahren nach Anspruch 1, **dadurch gekennzeichnet, dass** in Abhängigkeit der Positionsdaten ein Kanalelement (4) aus einem den Leitkanal (5) bereits umfassenden Kanalelemente-Rohling gefertigt wird, insbesondere durch Materialabtrag mit einer Bearbeitungsmaschine oder wenigstens teilweise durch generative Fertigung erstellt wird.

3. Verfahren nach einem der vorherigen Ansprüche, **dadurch gekennzeichnet, dass** die Bearbeitung des Kanalelement-Rohlings und/oder Montageelement-Rohlings mit einer 4-Achsen-Fräse erfolgt, in welche ein jeweiliger Rohling mittels eines am Rohling angeordneten Spannelementes (5a, 9) eingespannt wird.

4. Verfahren nach einem der vorherigen Ansprüche, **dadurch gekennzeichnet, dass** das Abdruckelement (1) mit einem Befestigungselement (2) an der Montageplatte (12) direkt oder indirekt befestigt wird, welches eine radiologische Referenz (3) umfasst, insbesondere in Form von Metallelementen (3), insbesondere Metallstiften (3), die in korrespondierende Ausnehmungen / Bohrungen direkt oder indirekt an der Montageplatte (12) eingreifen.

5. Verfahren nach einem der vorherigen Ansprüche, **dadurch gekennzeichnet, dass** mittels tomografischer Bildgebungstechnik, insbesondere digitaler Volumentomographie softwarebasiert ein virtuelles Kiefermodell eines Patienten erzeugt wird und ein virtueller Leitkanal relativ zum virtuellen Kiefermodell und zur radiologischen Referenz positioniert wird, wobei aus den Positionsdaten des virtuellen Leitkanals Positionsdaten gebildet werden, welche die Position der formschließenden Montagestruktur (10) am Montageelement (6) bestimmen.

6. Verfahren nach einem der vorherigen Ansprüche, **dadurch gekennzeichnet, dass** die formschließende Montagestruktur (10) am Montageelement (6) und/oder die formschließende Struktur (14) an der Montageplatte (12) als sich verjüngende, in wenigstens zwei Richtungen periodisch nebeneinander angeordnete Vorsprünge ausgebildet ist, insbesondere als pyramidenförmige Vorsprünge mit wenigstens drei, bevorzugt vier zueinander geneigten Seitenflächen oder als kegelförmige Vorsprünge.

## Claims

1. Method for producing a dental drilling template which is adapted to a jaw structure of a patient and comprises at least one guide channel (5) for guiding a drilling tool, in which the guide channel (5) is arranged in accordance with positional data on an impression element (1) comprising a negative impression of the jaw structure, wherein
a. a mounting element (6), which comprises an adaptation element (7) and is adapted to the required position of the guide channel (5), is produced by machining a material by means of a machining machine,
b. wherein, during production, a mould-closing mounting structure (10), preferably a mounting structure (10) that is periodically mould-closing in at least two directions, is produced on the mounting element (6) in accordance with the positional data,
c. a channel element (4) already comprising the guide channel (5), in particular in the form of a metal sleeve, and comprising an adaptation area which corresponds to the adaptation element (7) of the mounting element (6), and which is in particular formed by the guide channel (5) itself, is fixed on the mounting element (6) produced,
d. the assembly formed by the mounting element (6) and the channel element (4) comprising the mould-closing mounting structure (10) of the mounting element (6) is fixed at a location calculated in accordance with the positional data on a mounting plate (12) comprising a corresponding mould-closing structure (14), in particular a structure (14) that is periodic in at least two directions,
e. the impression element (1) is fixed with a fixing element (2) opposite the mounting plate (12) and
f. the channel element (4) is arranged on the impression element (1) by fixing, in particular by adhesive fixing on the impression element (1) and detaching from the mounting element (6).

2. Method according to Claim 1, **characterized in that**, in accordance with the positional data, a channel element (4) is produced from a channel-element blank already comprising the guide channel (5), in particular is created by removing material with a machining machine or at least partially by generative production.

3. Method according to one of the preceding claims, **characterized in that** the machining of the channel-element blank and/or mounting-element blank is performed with a 4-axis milling cutter, in which a respective blank is clamped by means of a clamping element (5a, 9) arranged on the blank.

4. Method according to one of the preceding claims, **characterized in that** the impression element (1) is fixed directly or indirectly on the mounting plate (12) with a fixing element (2), which comprises a radiological reference (3), in particular in the form of metal elements (3), in particular metal pins (3), which engage in corresponding clearances/holes directly or indirectly on the mounting plate (12).

5. Method according to one of the preceding claims, **characterized in that** a virtual jaw model of a patient is produced on a software basis by means of tomographic imaging technology, in particular digital volume tomography, and a virtual guide channel is positioned in relation to the virtual jaw model and the radiological reference, positional data that determine the position of the mould-closing mounting structure (10) on the mounting element (6) being formed from the positional data of the virtual guide channel.

6. Method according to one of the preceding claims, **characterized in that** the mould-closing mounting structure (10) is formed on the mounting element (6) and/or the mould-closing structure (14) is formed on the mounting plate (12) as tapering projections arranged next to one another periodically in at least two directions, in particular as pyramidal projections comprising at least three, preferably four, side faces inclined in relation to one another or as conical projections.

## Revendications

1. Procédé de fabrication d'un gabarit de perçage à usage dentaire adapté à une structure de mâchoire d'un patient, le gabarit de perçage comprenant au moins un conduit de guidage (5) pour le guidage d'un outil de perçage, selon lequel le conduit de guidage (5) est disposé, en fonction de données de position, sur un élément d'empreinte (1) comprenant une empreinte négative de la structure de mâchoire, dans lequel
a. un élément de montage (6) comprenant une pièce d'ajustement (7) et adapté à la position requise du conduit de guidage (5) est fabriqué par usinage de matière au moyen d'une machine d'usinage,
b. une structure de montage (10) à complémentarité de forme, de préférence une structure de montage (10) à complémentarité de forme périodique dans au moins deux directions, étant produite sur l'élément de montage (6) pendant la fabrication en fonction des données de position,
c. un élément de conduit (4) comportant déjà le conduit de guidage (5), en particulier sous la forme d'une douille métallique, et comportant une zone d'ajustement correspondant à la pièce d'ajustement (7) de l'élément de montage (6), laquelle zone d'ajustement est en particulier formée elle-même par le conduit de guidage (5), est fixé à l'élément de montage (6) fabriqué,
d. l'appariement de l'élément de montage (6) et de l'élément de conduit (4) fixés l'un à l'autre est fixé, par la structure de montage (10) à complémentarité de forme de l'élément de montage (6), en un point calculé en fonction des données de position, à une plaque de montage (12) comprenant une structure (14) correspondante à complémentarité de forme, en particulier une structure (14) périodique dans au moins deux directions,
e. l'élément d'empreinte (1) est fixé en regard de la plaque de montage (12) à l'aide d'un élément de fixation (2) et
f. l'élément de conduit (4) est disposé sur l'élément d'empreinte (1) par fixation, en particulier fixation par adhérence, à l'élément d'empreinte (1) et détachement de l'élément de montage (6).

2. Procédé selon la revendication 1, **caractérisé en ce qu'**en fonction des données de position, un élément de conduit (4) est fabriqué à partir d'une ébauche d'élément de conduit comportant déjà le conduit de guidage (5), en particulier par enlèvement de matière à l'aide d'une machine d'usinage ou au moins partiellement par fabrication additive.

3. Procédé selon l'une quelconque des revendications précédentes, **caractérisé en ce que** l'usinage de l'ébauche d'élément de conduit et/ou de l'ébauche d'élément de montage s'effectue à l'aide d'une fraiseuse à 4 axes, dans laquelle une ébauche respective est serrée au moyen d'un élément de serrage (5a, 9) disposé sur l'ébauche.

4. Procédé selon l'une quelconque des revendications précédentes, **caractérisé en ce que** l'élément d'empreinte (1) est fixé directement ou indirectement à la plaque de montage (12) à l'aide d'un élément de fixation (2), lequel élément d'empreinte comporte une référence radiologique (3), en particulier sous la forme d'éléments métalliques (3), en particulier de goupilles métallique (3), qui viennent en prise dans des évidements/alésages correspondants directement ou indirectement sur la plaque de montage (12).

5. Procédé selon l'une quelconque des revendications précédentes, **caractérisé en ce qu'**un modèle de mâchoire virtuel, basé sur un logiciel, d'un patient est produit au moyen d'une technique d'imagerie tomographique, en particulier d'une tomographie volumique numérisée, et un conduit de guidage virtuel est positionné par rapport au modèle de mâchoire virtuel et par rapport à la référence radiologique, et, à partir des données de position du conduit de guidage virtuel, des données de position étant formées, lesquelles déterminent la position de la structure de montage (10) à complémentarité de forme sur l'élément de montage (6) .

6. Procédé selon l'une quelconque des revendications précédentes, **caractérisé en ce que** la structure de montage (10) à complémentarité de forme sur l'élément de montage (6) et/ou la structure (14) à complémentarité de forme sur la plaque de montage (12) sont réalisées sous forme de saillies se rétrécissant et juxtaposées périodiquement dans au moins deux directions, en particulier sous forme de saillies en forme de pyramides dotées d'au moins trois, de préférence de quatre faces latérales inclinées les unes vers les autres ou sous forme de saillies coniques.
